# EUROPÄISCHE PATENTSCHRIFT

(11) **EP 0 708 640 B1**
(45) Veröffentlichungstag und Bekanntmachung des Hinweises auf die Patenterteilung: **16.09.1998**
(21) Anmeldenummer: 94918755.3
(22) Anmeldetag: 28.06.1994
(51) Int. Cl.: A61K 31/15, A61K 31/155, A61K 31/42

(54) **PHARMAZEUTISCHE ZUBEREITUNGEN MIT EINEM WIRKSTOFF, DER MODIFIZIERTE AMIDINGRUPPEN ENTHÄLT**
PHARMACEUTICAL PREPARATIONS WITH AN ACTIVE PRINCIPLE CONTAINING MODIFIED AMIDIN GROUPS
PREPARATIONS PHARMACEUTIQUES AVEC UN PRINCIPE ACTIF CONTENANT DES GROUPES AMIDINE MODIFIES

(30) Priorität: 28.06.1993 DE 4321444
(43) Veröffentlichungstag der Anmeldung: 01.05.1996
(60) Teilanmeldung: 98103835.9
(73) Patentinhaber: Clement, Bernd, D-24106 Kiel (DE)
(72) Erfinder: Clement, Bernd, D-24106 Kiel (DE)
(74) Vertreter: Pfenning, Meinig & Partner
(86) Internationale Anmeldenummer: DE9400756
(87) Internationale Veröffentlichungsnummer: WO9501168

(56) Entgegenhaltungen:
- EP-A- 0 272 455
- EP-A- 0 352 832
- WO-A-93/16037
- DE-A- 2 426 878
- FR-A- 2 092 895
- US-A- 3 658 832
- US-A- 3 795 735
- ARZNEIMITTEL FORSCHUNG, Bd.35, Nr.7, 1985 Seiten 1009 - 1014 CLEMENT, B. ET AL 'AMIDOXIMES OF PENTAMIDIME: SYNTHESIS, TRYPANOCIDAL AND LEISHMANICIDAL ACTIVITY'
- JOURNAL OF MEDICINAL CHEMISTRY, Bd.18, Nr.4, 1975 Seiten 430 - 432 ROSS, W.J. ET AL 'ANTIPARASITIC NITROIMIDAZOLES. 9. SYNTHESIS OF SOME 2-(4-DIALKYLAMINOMETHYLSTYRYL)- AND 2-(4-AMIDINOSTYRYL)- 5-NITRO-1-VINYLIMIDAZOLES.'
- ARCHIV DER PHARMAZIE, Bd.325, Nr.1, 1992 Seiten 61 - 62 CLEMENT, B. ET AL 'REDUCTION OF AMIDOXIME DERIVATIVES TO PENTAMIDINE IN VIVO'
- INDIAN JOURNAL OF CHEMISTRY, Bd.22B, September 1983 Seiten 898 - 900 CHAUHAN, P.M.S. ET AL 'SYNTHESIS OF 2,8-DIAMIDINODIBENZ(b,f)OXEPIN & RELATED COMPOUNDS AS POTENTIAL LEISHMANICIDES'
- ARZNEIMITTEL FORSCHUNG, Bd.42, Nr.12, 1992 Seiten 1497 - 504 CLEMENT, B. ET AL 'METABOLIC N-HYDROXLATION OF DIMINAZENE IN VITRO'
- JOURNAL OF MEDICINAL CHEMISTRY, Bd.12, Nr.3, 1969 Seiten 381 - 383 FANSHAWE, W.J. ET AL '1,2,4-OXADIAZOLYLPYRIDINIUM SALTS. ORAL HYPOGLYCEMIC AGENTS'
- 'THE MERCK INDEX' 1989 , MERCK & CO. INC. , RAHWAY, N.J., U.S.A. ELEVENTH EDITION Siehe S.515, Nr.3256; S.815, Nr.5067 & S.1128, Nr.7071

## Beschreibung

Die vorliegende Erfindung betrifft eine neuartige pharmazeutische Zubereitung, enthaltend einen Pentamidin-Wirkstoff und deren Verwendung.

Um Pneumocyctis carinii Pneumonie, an der nahezu 70% aller Aids-Patienten ohne prophylaktische Maßnahmen während ihres Krankheitsverlaufes erkranken, zu therapieren, ist es bekannt, wäßrige Lösungen von Pentamidin-Diisethionat mit Hilfe von speziellen Verneblern in Aerosolform anzuwenden. Hierbei besitzt dieses bekannte Pentamidin-Diisethionat eine Struktur, wie sie nachfolgend durch die Formel P wiedergegeben ist.

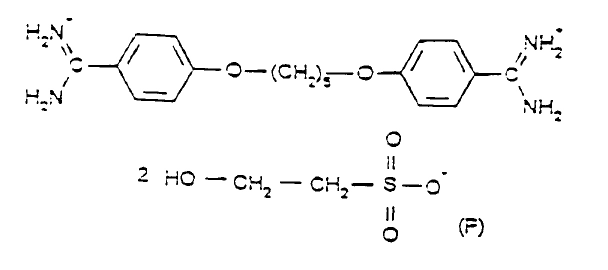

Das bekannte Pentamidin-Isethionat weist jedoch den Nachteil auf, daß es bei oraler Anwendung schlecht resorbiert wird und somit bei dieser Applikationsart keine pharmakologische Wirkung im Körper ausüben kann. Aus diesem Grund wurde das Präparat intramuskulär oder intravenös an die Patienten verabreicht. Die dazu vorhandenen galenischen Formulierungen weisen jedoch gravierende Nebenwirkungen auf, die auf die Applikationsform zurückzuführen sind.

Bei intravenöser Applikation können ein starker Blutdruckabfall (Hypotonie) verbunden mit Übelkeit und Erbrechen bis hin zur Ohnmacht auftragen. Die intramuskuläre Applikation bietet auch keine besonderen Vorteile, da häufig starke Schmerzen an der Einstichstelle bis hin zu Gewebsnerkrosen, die einer langwierigen Folgebehandlung bedürfen, beobachtet werden.

Bei der zuvor beschriebenen Vernebelung des Pentamidin-Diisethionat treten diese Nebenwirkungen nicht auf. Allerdings sind durch Vernebelung nur leichte bis mittelschwere Fälle von Pneumocyctis carinii Pneumonie zu behandeln, da eine schwere Erkrankung eine Inhalation des Aerosols unmöglich macht oder zumindestens erheblich erschwert. Des weiteren setzt diese Anwendungsform ein hohes Maß an Kooperation des Patienten voraus, weil der Patient die richtige Inhalationstechnik lernen muß, um eine gleichmäßige Wirkstoffverteilung zu erzielen, die letztendlich auch entscheidend für den Therapieerfolg ist.

Der geringen systemischen Belastung bei der Aerosoltherapie und der damit verbundenen geringeren Toxizität dieser Applikationsform stehen aber einige gravierende Nachteile gegenüber. So erfordert jede Anwendung einen ambulanten Krankenhausaufenthalt, bei der möglichst ein Arzt zugegen sein sollte, da bei der Aerosoltherapie häufig (bis zu 15% der Anwendungen) Bronchospasmen auftreten können, die ärztliche Gegenmaßnahmen erforderlich machen. Außerdem wird für die pulmonale Applikation ein spezieller Vernebler benötigt, der eine gleichförmige Teilchengröße von 0,5 bis 30 µm Durchmesser erzeugen kann.

Je nach Verneblertyp und je nach Art der Einstellung dieses Gerätes gelangen häufig nicht ausreichende Wirkstoffkonzentrationen an den vorgesehenen Wirkort.

Verlaufsformen mit extrapulmonarer Beteiligung können auf diese Art und Weise nicht therapiert werden. Damit verbunden ist die Rezidivrate von Pneumocyctis carinii Pneumonie bei nicht regelmäßiger ordnungsgemäßer prophylaktischer Anwendung der Aerosoltherapie erhöht.

Wie vorstehend dargelegt, zeigen Pentamidin enthaltene pharmazeutische Zubereitungen bei oraler Anwendung nahezu keine pharmakologische Wirkung. Die Voraussetzung für einen therapeutischen Effekt eines Wirkstoffes, nach oraler Gabe, stellt nämlich dessen Aufnahme aus dem Magen-Darm-Trakt dar. Der wichtigste Mechanismus eines solchen Membrandurchtritts ist dabei die passive Diffusion. Der Grad der Resorption auf dem Wege der passiven Diffusion ist nun dabei abhängig von der Lipopholie und damit eng in Zusammenhang stehend von der Acidität bzw. der Basizität des Wirkstoffes. Eine stark basische Verbindung wie das Pentamidin (pKₐ = 11,4) liegt im Magen (pH = 1) und im Darm (pH = 7,4) nahezu vollständig ionisiert vor. Das Molekül ist während der gesamten Magen-Darm-Passage hydrophil. Eine orale Resorption, die an den Durchtritt einer lipophilen Membran gebunden ist, erfolgt daher nur in einem sehr geringen Maße. Die starke Basizität des Pentamidin ist dabei auf seine funktionellen Gruppen, nämlich die Amidine zurückzuführen. Dies ist offensichtlich der Hinderungsgrund, warum bisher nur bei der oralen Anwendung eine völlig unzureichende Resorption und damit eine einhergehende nur sehr geringe pharmakologische Wirkung erfolgt.

Es ist zu vermuten, daß offensichtlich alle Wirkstoffe, die als funktionelle Gruppe ein Amidin aufweisen, eine ungenügende Resorption bei der oralen Anwendung zeigen.

Ausgehend hiervon ist es die Aufgabe der vorliegenden Erfindung, eine pharmazeutische Zubereitung vorzuschlagen, die einen Pentamidin-Wirkstoff enthält, welcher oral angewendet werden kann.

Diese Aufgabe wird durch eine pharmazeutische Zubereitung mit den kennzeichnenden Merkmalen des Patentanspruchs 1 gelöst. Die Unteransprüche geben vorteilhafte Weiterbildungen an. Die Verwendung ist durch die Merkmale der Ansprüche 3-11 gekennzeichnet.

Erfindungsgemäß wird demnach vorgeschlagen, daß die pharmazeutische Zubereitung den Pentamidin-Wirkstoff, der mindestens eine Amidin wirksame funktionelle Gruppe enthält in Form mindestens einer Verbindung der folgenden Formeln: 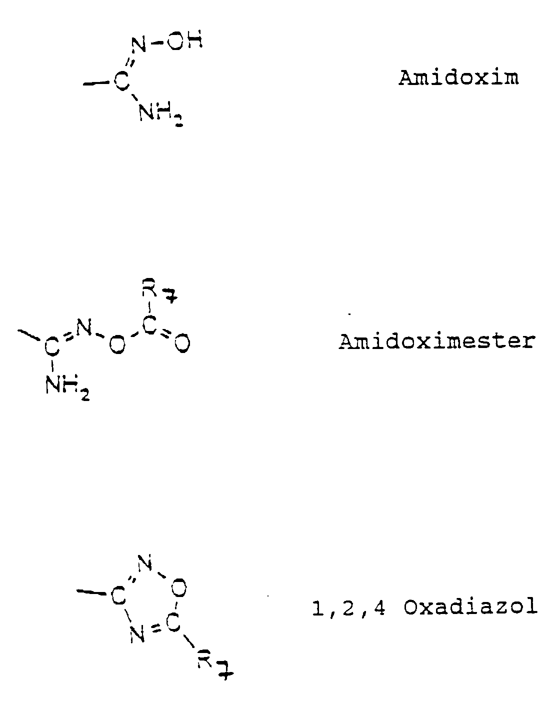 einzusetzen, wobei R₇ ein Alkylrest und/oder ein Arylrest ist. Amidoxime, Amidoximester und 1, 2, 4 Oxadiazole werden nun oral resorbiert und durch Esterasen und N-Reduktion wieder in die eigentlichen Wirkformen, die Amidine zurückverwandelt ("pro-drug"-Prinzip). Somit liegt für die orale Anwendung für Amidine eine optimale Arzneiform vor, die für die verschiedensten Indikationen einsetzbar ist.

Beispiele hier für sind:
- Prophylaxe und Therapie der viszeralen Leishmaniose und der kutanen Leishmamiose
- Prophylaxe und Therapie der Trypanosomiasis (afrikanische Schlafkrankheit,
- Prophylaxe und Therapie der durch Pneumocystis carinii verursachten Pneumonie (PcP).
- Hemmung von Proteasen, Thombininhibitoren, Fibrinogen-Rezeptor-Antagonisten, Plättchen-Aggregationshemmer
- Hemmung des Wachstums von malignen Tumoren (Krebschemotherapie)
- Blutdrucksenkung
- N-Methyl-D-Aspartat Rezeptor-Antagonisten und damit Neuroprotektion.

Die ausgezeichnete Resorbierbarkeit der abgewandelten Amidinfunktionen im Magen-Darmtrakt ist offensichtlich auf die stark verminderte Basizität und erhöhter Lipophilie der Wirkstoffmoleküle zurückzuführen. Wie bereits ausgeführt, ist für einen therapeutischen Effekt eines Wirkstoffes nach oraler Gabe die Aufnahme aus dem Magen-Darm-Trakt von Bedeutung, diese steht im engen Zusammenhang mit der Acidität bzw. Basizität des Wirkstoffes. Mit der chemischen Abwandlung der Amidinfunktion bis hin zum Amidoximester bzw. Oxadiazol wird jedoch die Basizität in einem sehr hohen Maße verringert. Der pKₐ-Wert von Amidin, der bei 11 liegt, sinkt bis zum Amidoximester bzw. Oxadiazol auf Werte unter 5. Der Prozentanteil der freien Base bei pH 7,4 steigt damit vom Amidin von Null bis zum Amidoximester bzw. Oxadiazol auf 100. Im Darm, dem Hauptresorptionsort für Wirkstoffe, liegt somit das Amidoxim bzw. der Amidoximester bzw. das Oxadiazol nahezu vollständig in Form der freien Base vor. Parallel zur Abnahme der Basizität nimmt durch die vorgenommene Abwandlung der Amidinfunktion die Lipophilie der entsprechenden Wirkstoffe zu. Die erfindungsgemäß vorgeschlagene pharmazeutische Zubereitung mit der abgewandelten Amidinfunktion weisen somit eine hervorragende orale Resorbierbarkeit auf und erhöhen damit deutlich die pharmakologische Wirkung des Amidins. Der vorgeschlagenen Wirkstoff ist nicht nur im Magen und/oder Darm resorbierbar, sondern sind auch in der Lage, die Blut-/Hirnschranke zu überwinden.

Erfindungsgemaß enthält die Zubereitung einen Pentamidin-Wirkstoff , der durch die nachfolgende allg. Formel 1 wiedergegeben ist.

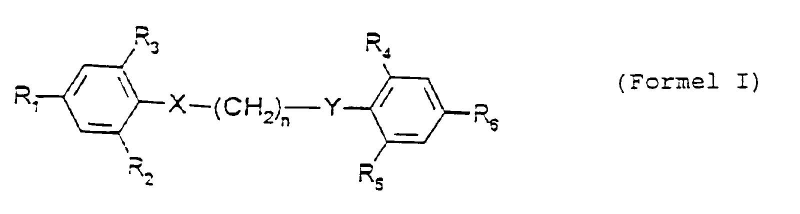

In der Formel I sind R₁ und R₆ gleich oder verschieden, und aus folgenden Gruppen ausgewahlt: 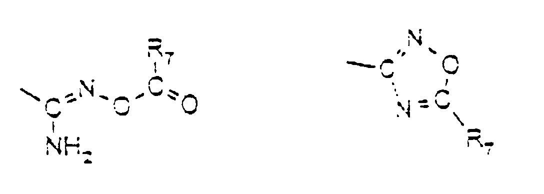 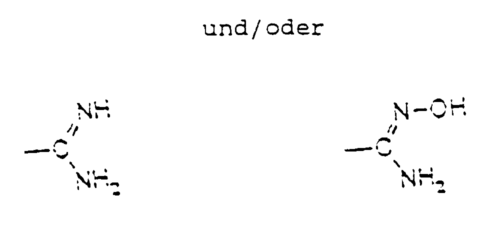 wobei R₇ Wasserstoff, ein Alkylrest und/oder ein Arylrest ist und deren Salze. Desweiteren sind in der Formel I R₂, R₃, R₄ und R₅ gleich oder verschieden und bedeuten Wasserstoff, eine -NO₂-Gruppe, Halogene und/oder eine OR₈-Gruppierung, wobei R₈ Wasserstoff und/oder eine Alkylgruppe sein kann. X und Y, die gleich oder verschieden sein können, stehen in der Formel I für Sauerstoff, Stickstoff oder Schwefel, während n eine ganze Zahl zwischen O und 8 bedeutet.

In der Formel I sind nicht gleichzeitig R₁ und R₆ gleich oder verschieden sind und die Gruppierungen 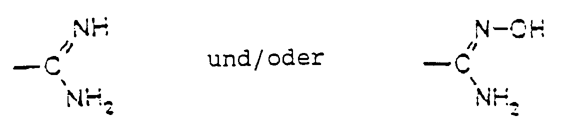 bedeuten, wenn n = 5 ist und
R₂ und R₅ Wasserstoff und wenn X und Y gleich sind und Sauerstoff bedeuten.

Überraschend konnte festgestellt werden, daß die zuvor genannte und den in Formel I wiedergegebenen Wirkstoff enthaltende erfindungsgemäße pharmazeutische Zubereitung ausgezeichnete prophylaktische und therapeutische Eigenschaften besitzt, wenn die erfindungsgemäße Zubereitung zur Prophylaxe und/oder Therapie von Pneumocyctis carinii Pneumonie eingesetzt wird. Insbesondere zeigte sich, daß der in Formel I wiedergegebene Wirkstoff nicht die eingangs in Verbindung mit dem bekannten Pentamidin-Isethionat beschriebenen Nachteile aufweist, so zum Beispiel auch dann keine Gewebenekrosen und Hypotensionen hervorruft, wenn die erfindungsgemäße pharmazeutische Zubereitung, die den in Formel I wiedergegebenen Wirkstoff aufweist, anders als durch Vernebelung appliziert wird. Eine derartige orale Applikation weist darüber hinaus noch den Vorteil auf, daß der in der erfindungsgemäßen pharmazeutischen Zubereitung enthaltene Wirkstoff besonders einfach und reproduzierbar dosiert werden kann, so daß dementsprechend auch die Prophylaxe und/oder Therapie bei Verwendung der erfindungsgemäßen pharmazeutischen Zubereitung erheblich verbessert ist. Eine vergleichbare Wirkung besitzen auch Salze des Wirkstoffes gemäß Formel I.

Die erfindungsgemäß Zubereitung zeichnet sich insbesondere dadurch aus, daß sie im Vergleich zum Pentamidin-Diisethionat eine größere Lipophilie und eine geringere Basizität besitzt, und somit oral resorbierbar und ZNS-gängig ist, was die Möglichkeit der Therapie und/oder Prophylaxe wesentlich verbessert.

Insbesondere dann, wenn der in Formel I wiedergegebene Wirkstoff, der in der erfindungsgemäßen pharmazeutischen Zubereitung enthalten ist, einen chemischen Aufbau aufweist, bei dem R₇ in den allgemeinen Formeln 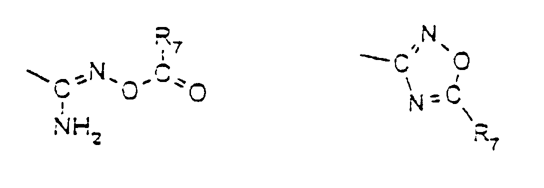 eine C₁-C₄-Alkylgruppe, insbesondere eine Methylgruppe, oder ein C₁₂-C₁₈-Alkylrest bedeutet, ist die pharmazeutische Wirksamkeit einer derartigen Zubereitung nochmals weiter verbessert, so daß dementsprechend die Konzentration des Wirkstoffes in der pharmazeutischen Zubereitung reduziert oder die täglich zu applizierende Dosierung verringert werden kann.

Wie bereits vorstehend in Verbindung mit der erfindungsgemäßen Zubereitung dargelegt ist, können in der Formel I R₁ und R₆ gleich oder verschieden sein. Insbesondere dann, wenn R₁ und R₆ in der wiedergegebenen Formel I gleich sind, läßt sich ein derartig aufgebauter Wirkstoff besonders einfach synthetisieren, da hierbei eine aufwendige Isolierung eines derartigen Wirkstoffes im Anschluß an die Synthese entfallen kann.

Wie bereits eingangs ausgeführt ist, kann in der allgemeinen Formel I n eine ganze Zahl zwischen O und 8 bedeuten. Vorzugsweis weist jedoch die erfindungsgemäße Zubereitung einen Aufbau auf, bei dem n für eine ganze Zahl zwischen 2 und 6 steht.

Eine weitere, vorteilhafte Weiterbildung der erfindungsgemäßen Zubereitung, die sich durch eine sehr hohe pharmazeutische Wirksamkeit und eine einfache Synthesemöglichkeit des Wirkstoffes der allgemeinen Formel I auszeichnet, sieht vor, daß in der Formel I R₂, R₃, R₄ und/oder R₅ eine OCH₃-Gruppe bedeuten.

Die Konzentration des Wirkstoffes in der erfindungsgemäßen pharmazeutischen Zubereitunge ist abhängig von dem jeweiligen Anwendungsfall sowie der täglichen Dosierung. Vorzugsweise weist die erfindungsgemäße Zubereitung den Wirkstoff gemäß Formel I in einer Konzentration zwischen 0,01 Gew.% und 50 Gew.% auf, insbesondere in einer Konzentration zwischen 1 Gew.% und 20 Gew.%.

Vorstehend ist im Zusammenhang mit der erfindungsgemäßen pharmazeutischen Zubereitung stets die Rede davon gewesen, daß die erfindungsgemäße Zubereitung den Wirkstoff enthält. Selbstverständlich ist es jedoch auch möglich, hier ein Gemisch von Wirkstoffen vorzusehen.

Bei der erfindungsgemäßen oralen Anwendung wird nun in besonderem Maße sichergestellt, daß der Wirkstoff reproduzierbar dosiert werden kann, was einen entscheidenden Einfluß auf die Therapie und Prophylaxe bei der Behandlung von Pneumocyctis carinii Pneumonie besitzt. Ferner ist durch die orale Anwendung sichergestellt, daß auch schwere Verlaufsformen mit extrapulmonarer Manifestation, die sich nicht oder nur schwer per Aerosol behandeln lassen, einer Therapie zugeführt werden können.

Überraschend konnte desweiteren festgestellt werden, daß die erfindungsgemäße pharmazeutische Zubereitung nicht nur zur Prophylaxe und/oder zur Therapie von Pneumocyctis carinii Pneumonie, sondern auch zur Prophylaxe und/oder zur Therapie von tierischen oder menschlichen Trypanosomeninfektionen und/oder von Leishmaniose eingesetzt werden kann. Auch hierbei bietet sich eine orale Applikation der erfindungsgemäßen pharmazeutischen Zubereitung an, wobei bei dieser oralen Applikation überraschenderweise nicht die Nebenwirkungen auftreten, wie diese eingangs unter Hinweis auf das Pentamidin-Isethionat beschrieben sind.

Diese zuvor beschriebene hohe Wirksamkeit der erfindungsgemäßen Zubereitung wird darauf zurückgeführt, daß der in der Formel I wiedergegebene Wirkstoff aufgrund seiner geringeren Basizität und höheren Lipophilie im Vergleich zum Pentamidin-Diisethionat bei der oralen Applikation im Laufe der Magen-Darm-Passage im hohen Maße resorbiert wird. Während oder nach erfolgter Resorption findet im Körper eine Metabolisierung hin zum entsprechenden Diamidin bzw. Diamidinderivat statt, das aufgrund seiner Hydrophilie bei einer oralen Applikation nicht oder nur geringfügig im Magen-Darm-Trakt resorbiert würde, andererseits aber die erwünschte pharmazeutische Wirksamkeit besitzt.

Die zuvor kurz beschriebene und in vivo ablaufende Metabolisierung der erfindungsgemäßen pharmazeutischen Zubereitung läßt sich schematisch durch das nachfolgend wiedergegebene Schema am Beispiel von Ausführungsvarianten der erfindungsgemäßen pharmazeutischen Zubereitung wiedergeben.

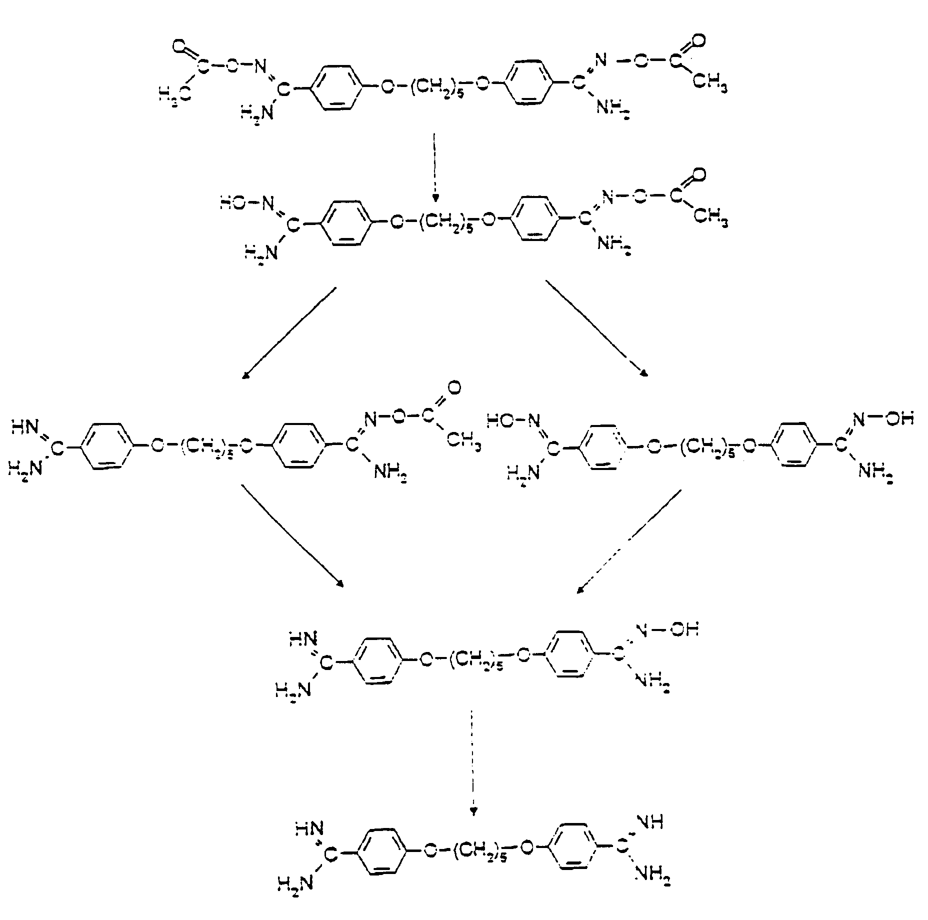

Eine besonders geeignete Ausführungsform der erfindungsgemäßen pharmazeutischen Zubereitung weist einen Wirkstoff auf, wie dieser durch die nachfolgend wiedergegebenen Formel II, III oder IV charakterisiert ist.

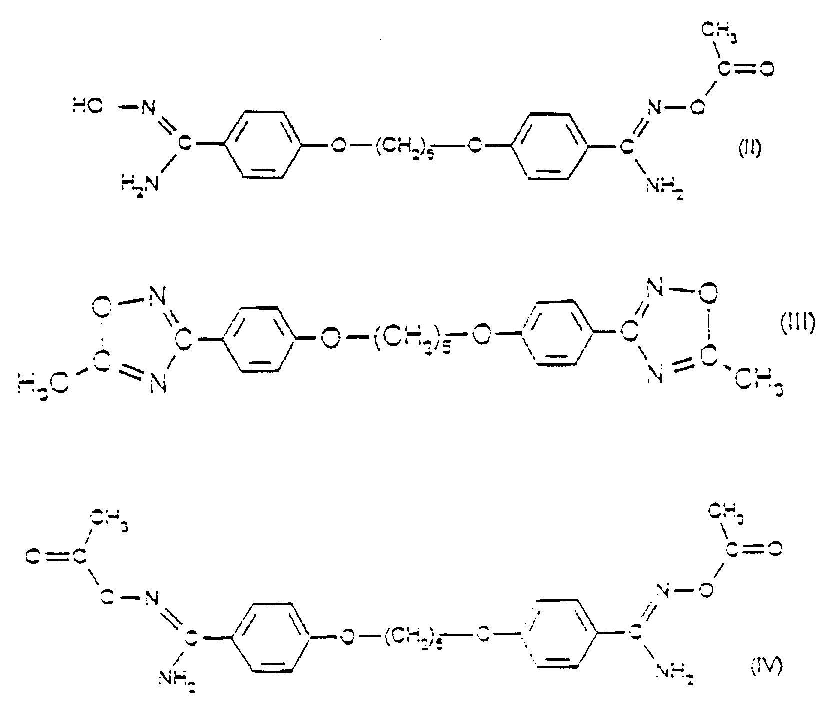

Diese speziellen Ausführungsformen der erfindungsgemäßen Zubereitung, die die Wirkstoffe gemäß der vorstehenden Formeln II bis IV aufweisen, können mit hervorragendem Erfolg oral bei der Behandlung und/oder Prophylaxe von Pneumocyctis carinii Pneumonie und/oder Leishmaniose, Trypanosomeninfektionen und/oder als Cytostatika eingesetzt werden.

Grundsätzlich ist zu dem in der erfindungsgemäßen Zubereitung enthaltenen Wirkstoff, z.B. der durch die allgemeinen Formeln I bis IV wiedergegeben ist, anzumerken, daß die verbesserte pharmazeutische Wirkung der beanspruchten erfindungsgemäßen Zubereitung u.a. mit darauf zurückgeführt wird, daß die erfindungsgemäße Zubereitung offensichtlich in der Lage ist, die Blut-Hirn-Schranke zu überwinden. Dies wiederum bewirkt, daß mit der erfindungsgemäßen Zubereitung, insbesondere auch mit der zuvor beschriebenen Ausführungsform, die einen Wirkstoff gemäß der Formeln II bis IV beinhaltet, auch Erreger erfolgreich bekämpft werden können, die das zentrale Nervensystem des Patienten befallen haben, was mit dem bekannten Pentamidin-Isethionat nicht möglich ist.

Somit steht durch die erfindungsgemäße pharmazeutische Zubereitung ein wirksames Mittel zur Behandlung zentralnervöser Verlaufsformen von Trypanosomeninfektionen zur Verfügung, das nicht die hohe Toxizität der bisher hier eingesetzten arsenhaltigen Therapeutika besitzt.

Die orale Darreichungsform kann als flüssige, halbfeste oder feste Zubereitung, insbesondere als Tablette, Dragee, Pellets oder Mikrokapseln, aufgemacht sein. Hierbei werden für solche Ausführungsformen, bei denen flüssige Zubereitungen eingesetzt werden, der Wirkstoff bzw. das Wirkstoffgemisch in einem geeigneten, nicht toxischen Lösungsmittel, wie beispielsweise Wasser, einwertige Alkohole, insbesondere Ethanole, mehrwertige Alkohole, insbesondere Glycerin und/oder Propandiol, Polyglykole, insbesondere Polyethylenglykole und/oder Miglyol, Glycerinformal, Dimethylisosorbit, natürliche und/oder synthetische Öle und/oder Ester aufgenommen.

Für die Herstellung von halbfesten oder festen Zubereitungen gelangen die üblichen Grundmassen, wie beispielsweise Bentonit, Veegum, Guarmehl und/oder Cellulosederivate, insbesondere Methylcellulose und/oder Carboxymethylcellulose, sowie Polymere aus Vinylalkohole und/oder Vinylpyrrolidone, Alginate, Pektine, Polyacrylate, feste und/oder flüssige Polyethylglykole, Paraffine, Fettalkole, Vaseline und/oder Wachse, Fettsäuren und/oder Fettsäureester zur Anwendung. Desweiteren können in festen Zubereitungen die an sich bekannten Streckmittel, wie beispielsweise kolloidale Kieselsäure, Talkum, Milchzucker, Stärkepulver, Zucker, Gelatine, Metalloxide und/oder Metallsalze enthalten sein. Als weitere Zusatzstoffe bieten sich Stabilisatoren, Emulgatoren, Dispergatoren sowie Konservierungsstoffe an.

Vorteilhafte Weiterbildungen der erfindungsgemäßen Zubereitung sind in den Unteransprüchen angegeben.

Die erfindungsgemäß Zubereitung wird nachfolgend anhand von Ausführungsbeispielen näher erläutert.

### Ausführungsbeispiel 1

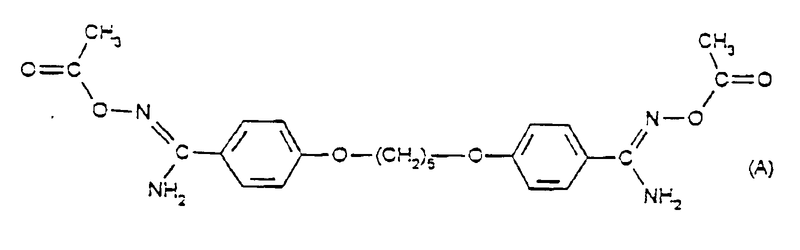

1 g 4,4'-Pentamethylendioxydibenzamidoxim wurden langsam mit ca. 10 ml frisch destilliertem Acetanhydrid versetzt und eine Stunde lang bei Raumtemperatur rühren gelassen. Zum Ende der Reaktion wurde das überschüssige Acetanhydrid mit Wasser hydrolisiert. Der entstandene Niederschlag wurde abgefrittet und mit 5 ml 3n Ammoniaklösung gewaschen. Der so behandelte Niederschlag wurde so lange mit demineralisiertem Wasser gewaschen, bis keine Ammoniakgeruch mehr wahrnehmbar war. Abschließend wurde der Niederschlag 1 Stunde lang bei 100° C im Trockenschrank getrocknet und aus Acetonitril umkristrallisiert.
- Ausbeute:: 590 mg (48% der theoret. Ausbeute), feine farblose Nadeln
- Schmp.:: 152° C
- IR-Daten KBr- Preßling) in cm^{-1:}: 3500 (NH), 1752 (COOR), 1620 (C=N)
- ¹H-NMR-Daten 400 MHZ-Spektrum ([D₆]-DMSO) in ppm:: 1,61 l (quint, 2H, -CH₂-); 1,81 (quint, 4H, 2-CH₂-); 2,15 (s,6H, 2 -CO-CH₃), 4,05 (t, 4H, 2 CH₂-O); 6,7 (s, 4H, 2 HN₂); 7,34 (mc, AA' BB', 8 H, Aromaten-H)
- ¹³C-NMR-Daten 400 MHZ-Spektrum ([D₆]-DMSO):: 21,47 (-CO-CH₃); 23,77 (-CH₂-); in ppm 29,94 (-CH₂-); 69,13 (-O-CH₂);, 115,71 (c-3 c-3'); 125,18 (-C=N-); 129,72 (c-2, c-2'); 157,69 (C-4); 161,95 (C-1); 170,15 (-O-CO-CH₃)
- C₂₃H₂₈O₆N₄(456,45): Ber. C 60,52% H 6,18% N 12,27%
Gef. C 60,58% H 6,15% N 12,65%

### Ausführungsbeispiel 2

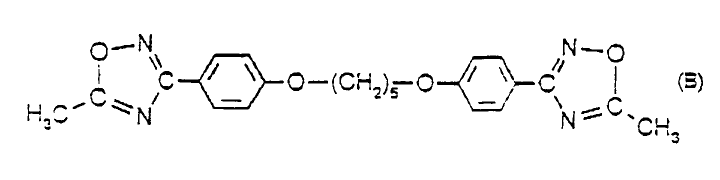

1 g 4,4'-Pentamythylendioxydibenzamidoxim wurden mit 10 ml frisch destilliertem Acetanhydrid versetzt und 5 Minuten unter Rückfluß erhitzt. Nach dem Abkühlen und der Hydrolyse des überschüssigen Acetanhydrids wurde ein schwach gelb gefärbtes Rohprodukt erhalten, das säulenchromatographisch (Kieselgel 60 - CHCl₃/MeOH (V:V, 9:1) gereinigt und anschließend aus absolutem Ethanol umkristallisiert wurde.
- Ausbeute:: 677 mg (60% der theroret. Ausbeute), feine farblose Nadeln
- Schmp.:: 137° C
- IR-Daten (KBr- Preßling) in cm^{-1:}: 1620 (C=N), 1275 (C-O-C)
- ¹H-NMR-Daten 400 MHZ-Spektrum (CDC13) in ppm:: 1,69 (quint, 2H, -CH₂-); 1,90 (quint, 4H, -CH₂-); 2,63 (s, 6H, -CH₃), 4,06 (t, 4H, -O-CH₂-), 7,48 (mc, AA' BB', 8 Aromaten H)
- ¹³C-NMR-Daten 400 MHZ-Spektrum (CDCl₃) in ppm:: 12,31 (-CH₃); 22,65 (-CH₂); 28,37 (-CH₂); 67,78 (-OCH₂-) 114,69 C-3, C-3'); 119,11 (-C=N-); 128,88 (C-2, C-2'); 161,30 (C-4); 168,4 (C-1); 176,18' (CH₃-C^{N}O)
- C₂₃H₂₄O₄N₄: Ber. C 65,70% H 5,75% N 13,32%
Gef. C 65,91% H 5,72% N 13,71%

### Ausführungsbeispiel 3

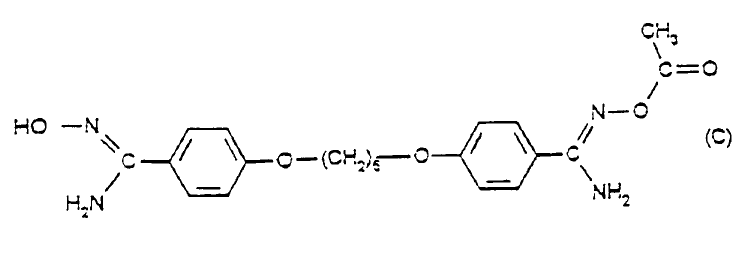

1 g Diacetyl-4,4'-Pentamethylendioxyddibenzamidoxim wurden in 100 ml eines Lösungsmittelgemisches, bestehend aus Chloroform-Methanol im Volumenverhältnis 9:1, gelöst und mit 5 g Schweinepankreas-Lipase (PPL) (Fa. Sigma Typ crude) versetzt und bei Raumtemperatur rühren gelassen. Die Reaktion wurde dünnschichtchromatographisch (Kieselgel -CHCl₃/MeOH (9:1)) verfolgt und abgebrochen, sobald sich Pentamethylendioxydibenzamidoxim gebildet hatte. Hiernach wurde die Lipase abfiltriert und das klare Filtrat mit Oxalsäure gesättigt und mit dem drefachen Volumen Ether versetzt. Der Ansatz wurde über Nacht im Kühlschrank stehen gelassen. Das ausgefallene Produkt wurde in Chloroform/Methanol (V:V 9:1) gelöst und säulenchromatographisch (Kieselgel, Chloroform/Methanol (V:V - 9:1) gereinigt.
- Ausbeute:: 40 mg (4,4% der theoret. Ausbeute)
- Schmp.:: 110° C
- IR-Daten(KBr- Preßling) in cm⁻¹:: 3510 (NH), 3375 (NH), 3200 (OH), 1750 (CH₃-COOR), 1670 (C=N-OH), 1620 (C=N-O-COCH₃)
- ¹H-NMR-Daten ([D₆]-DMSO) in ppm:: 1,60 (quint, 2H; CH₂), 1,82 (quint, 4 H, CH₂), 2,15 (s, 3 H, -COCH₃); 4,06 (t, 4 H, -O-CH₂-); 6,65 (s, 2 H, NH₂); 6,71 (s, 2 H, NH₂); 7,32 (mc,AA'BB', 4 H, Aromaten H), 7,34 (mc, AA'BB', 4 H, Aromaten H); 10-12 (bs, 1 H, OH)
- ¹³C-NMR-Daten ([D₆]-DMSO) in ppm:: 19,90 (CH₃-); 22,18 (-CH₂-); 28,23, (-CH₂-); 67,54 (-O-CH₂-); 67,62 (-O-CH₂-); 114,13 (C3', C3''); 114,32 (C3, C3'), 122,31 (C=N-OH); 122,59 (C=N-OCOR); 127,86 (C2', C2''); 128,13 (C2, C2'); 156,10 (C4); 160 (C4'); 160,51 (C1); 162,57 (C1'); 168,56 (O-COCH₃)
- C₂₁H₂₆O₅N₄(414,45) :: Ber. C 60,85% H 6,27% N 13,51
Gef. C 60,51% H 6,20% N 13,91%

Aufgrund der zuvor wiedergegebenen Analysedaten läßt sich feststellen, daß die nach den Ausführungsbeispielen 1 bis 3 hergestellten Produkte einen chemischen Aufbau aufweisen, wie dieser vorstehend durch die Formel A, die Formel B und die Formel C wiedergegeben ist.

Zum Nachweis der pharmazeutischen Wirksamkeit wurden die vorstehend beschriebenen Produkte bezüglich ihrer Wirkung gegenüber Pneumocyctis carinii untersucht.

Hierzu wurden weibliche Spraque-Dawley Ratten mit einem Durchschnittsgewicht von 200 bis 220 g 8 Wochen lang medikiertes Trinkwasser, das 1,5 mg Dexamethason und 10 mg Ofloxazin pro Liter enthielt, verabreicht. Am Ende dieser Prämedikation wurde bei einem Teil der Tiere eine Bronchiallavage unter Nembutalnarkose vorgenommen, um festzustellen, ob eine Infektion mit Pneumocyctis carinii vorlag. Hierzu wurden das Lavagematerial bei 3.000 U/min. zentrifugiert. 10 µl des resuspendierten Pelltes wurden auf einen Objektträger aufgetropft und an der Luft trocknen gelassen. Die so vorbereiteten Präparate wurden einer Giemsa-Schnellfärbung unterworfen und mikroskopisch untersucht. Als eine Infektion mit Pneumocyctis carinii festggestellt wurde, konnte mit der Wirkstofftestung begonnen werden.

Die Tiere wurden 10 Tage lang mit den zu testenden Wirkstoffen behandelt. Am Ende dieser Testzeit wurde bei den Tieren eine Bronchiallavage unter Nembutalnarkose vorgenommen. Diese Lavageflüssigkeit wurde mit Hilfe des Pneumocyctis-Direktnachweis-Tests der Firma Progen (Progen Biotechnik GmbH, Heidelberg) aufgearbeitet. Bei diesem Test reagieren fluoreszeinisothiocyanatmarkierte monoklonale Antikörper mit Pneumocyctis carinii in verschiedenen Entwicklungsstadien und lassen sich so im Fluoreszenzmikroskop sichtbar machen. Entsprechend einer Bewertungsskala, die in sechs Abstufungen von 0 bis 100 reicht, wurde mikroskopisch der Grad der Infektion der jeweiligen Lavageflüssigkeit bestimmt.

In der Tabelle 1 ist das Ergebnis der Verbindung 1 (Ausführungsbeispiel 1, Formel A)im Vergleich zu dem bekannten Pentamidin-Isethionat dargestellt.

**Tabelle 1**

| Auswertung der Testung auf Wirksamkeit gegen Pneumocyctis carinii | | |
|---|---|---|
| Nr. d.getest. Subst. | Tieranzahl | mikroskop. Auswertung |
| 1 | 9 | ++, ++, +, +, +-, +-, +-, +-, (+) |
| 2 | 11 | -, (+), ++, +, ++, +, (+), +-, +, ++, +- |
| 3 | 10 | +, ++, +-, +-, +, +-, ++, +++, +++, ++ |
| 4 | 11 | ++, +++, ++, ++, +++, ++, +++, +++, +++, +++ |
| 5 | 11 | ++, +++, ++, ++, +, ++, +, +, ++, +, + |
| 6 | 19 | ++, ++, +++, ++, ++, ++, +-, +, ++, ++, ++, +, ++, ++, ++, +++, +++, +, +++ |
| 7 | 10 | -, (+), -, -, -, -, (+), -, -, - |

1 = Diacetyl-4,4'-Pentamethylendioxidbenzamidoxim; 20 mg/kg - oral (vorgelöst in 5%igem DMSO und mit 0,04- iger Tylose aufgefüllt; Formel A, Ausführungsbeispiel 1).
2=Pentamidin-Isethionat in Aqua dest.; 20 mg/kg - i.m.
3=Pentamidin-Isethionat in Aqua dest.; 40 mg/kg - i.m.
4=Pentamidin-Isethionat in Aqua dest.; 20 mg/kg - oral
5=Pentamidin-Isethionat in Aqua dest.; 40 mg/kg - oral
6=unbehandelte Tiere, immunsupprimiert durch Dexamethason-Prämedikation
7=unbehandelte Tiere, nicht immunsupprimiert

| Bewertungsschema: | | |
|---|---|---|
| +++ | = | 100 Infektion |
| ++ | = | 75 |
| + | = | 50 |
| +- | = | 25 |
| (+) | = | 10 |
| - | = | 0 |

| Nr. d. gest. Subst. | Tier Anzahl | Mittelwert | Standard-abw. | Reduktion (in %) |
|---|---|---|---|---|
| 1 | 9 | 40 | 24 | 44,6 |
| 2 | 11 | 45 | 25 | 37,7 |
| 3 | 10 | 60 | 29 | 16.9 |
| 4 | 11 | 82 | 30 | ---- |
| 5 | 11 | 66 | 17 | 8,7 |
| 6 | 19 | 72 | 19 | ---- |
| 7 | 10 | 2 | 4 | ---- |

Wie aus der Tabelle 1 zu ersehen ist, zeigt die Verbindung 1 eine wesentlich größere Wirksamkeit gegenüber Pneumocyctis carinii als das bekannte Pentamidin-Isethionat. Besonders bemerkenswert ist, daß die Verbindung 1 bei einer oralen Applikation eine hohe Wirksamkeit besaß, während das bekannte PentamidinIsethionat nur bei einer parentaralen Applikation überhaupt einen therapeutischen Effekt zeigte.

Um die Wirksamkeit der vorstehend wiedergegebenen Verbindung 1 (Ausführungsbeispiel 1, Formel A) gegenüber Typanosomeninfektionen zu belegen, wurde der nachfolgend wiedergegebene Test durchgeführt. Hierbei wurde die Wirksamkeit gegen die tierpathogenen Erreger Trypanosoma brucei, Trypanosoma vivax, Trypanosoma congolense und Trypanosoma evansi sowie gegen den Erreger der menschlichen Schlafkrankheit, Trypanosoma Rhodesiense, untersucht.

Getestet wurde in einem Mäusemodell, wobei das bekannte Pentamidin-Isethionat als Vergleichssubstanz diente. Den mit dem jeweiligen Trypanomenstammen infizierten Tiere wurde die zu testende Substanz subcutan appliziert. Zur Kontrolle blieb die gleiche Anzahl infizierter Tiere unbehandelt. Nach einer Beobachtung von 3 Wochen wurde die Anzahl der geheilten Tiere, die Dosis curativa (D.C.), die Rezidivdosis (R.D.) und die Hemmdosis (H.D.) bestimmt. In den Tabellen 2 bis 6 sind exemplarisch die Ergebnisse dieser Tests wiedergegeben, wobei weitere detaillierte Angaben zu den eingesetzten Testmethoden ebenfalls in diesen Tabellen zu finden sind.

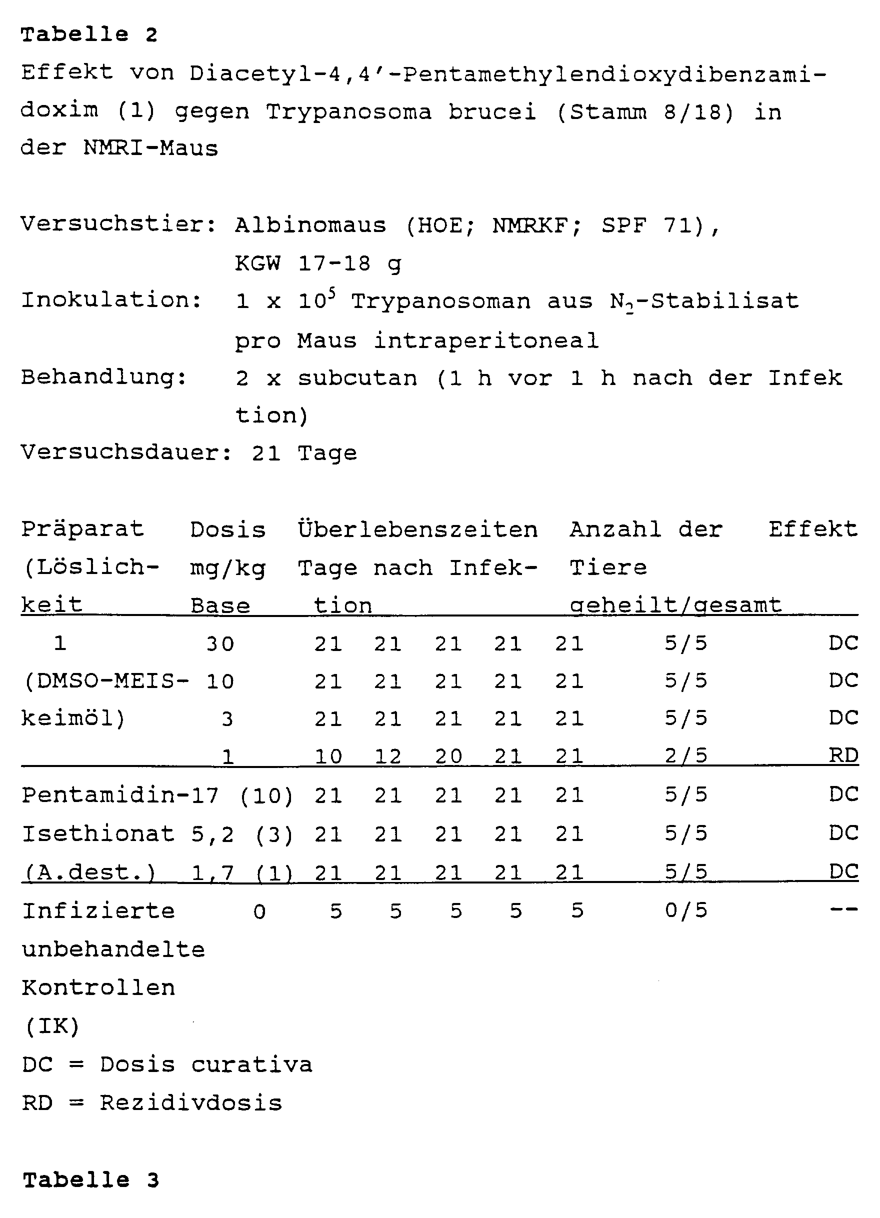

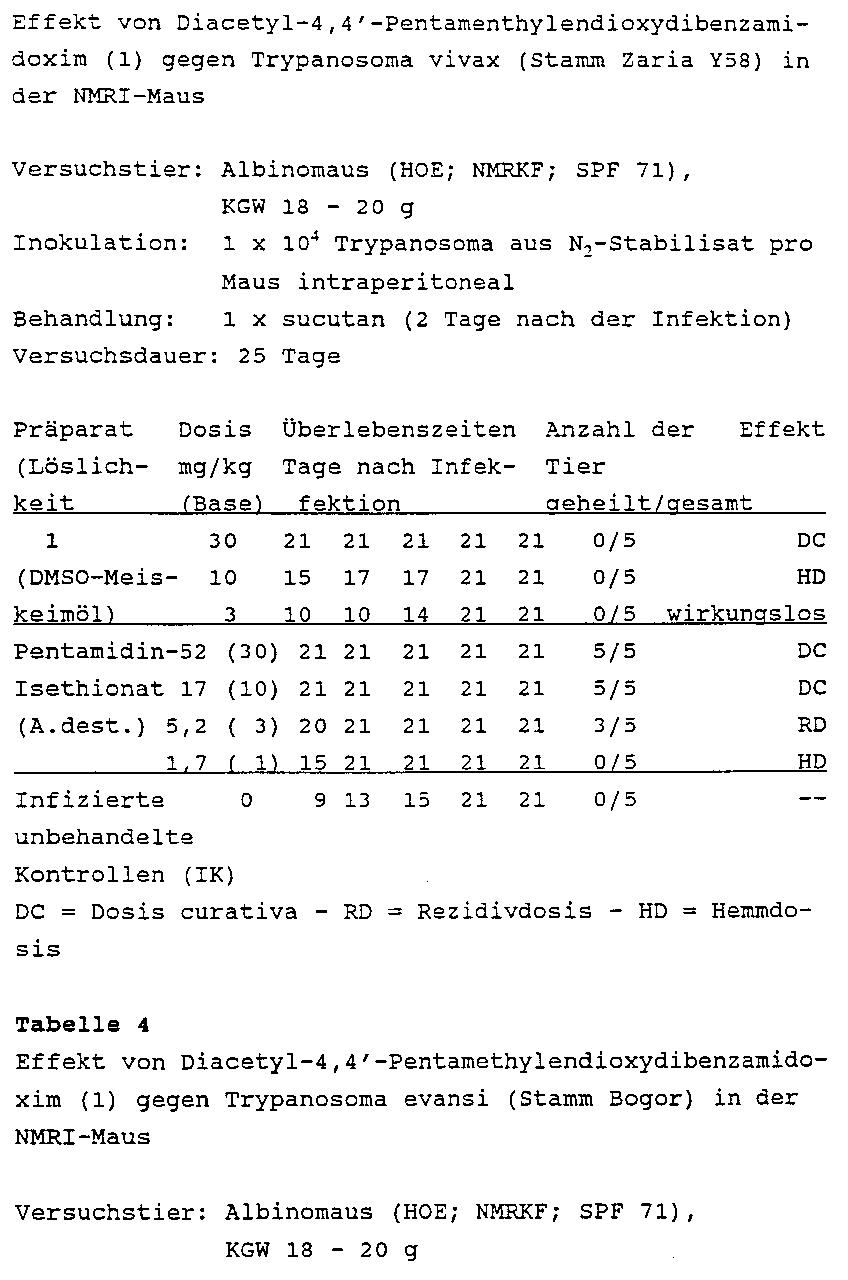

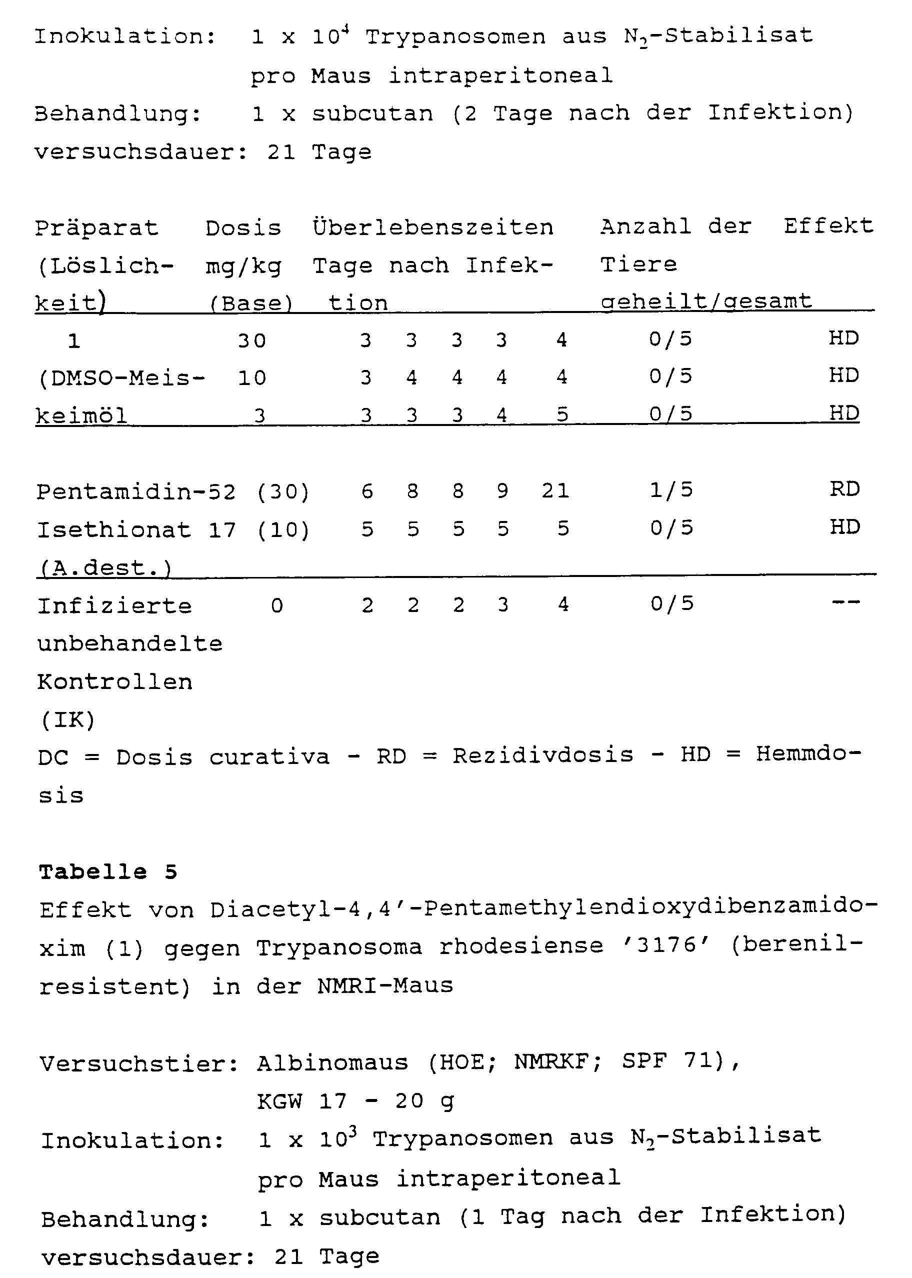

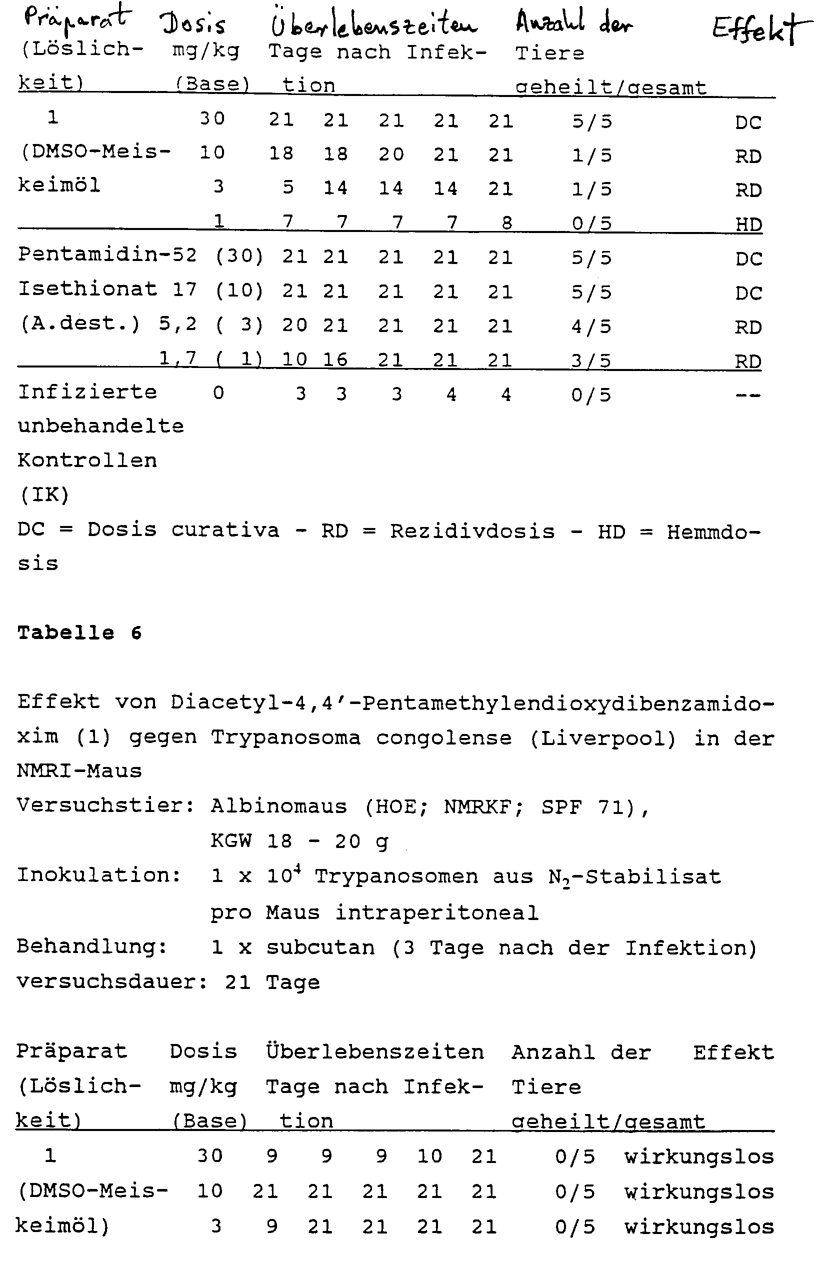

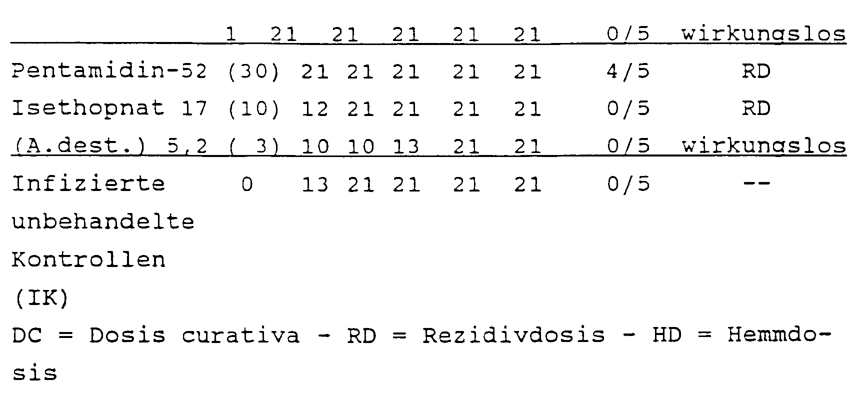

Wie den Tabellen 2 und 6 zu entnehmen ist, zeigt die Verbindung 1 eine gute Wirksamkeit gegenüber dem Erreger Trypanosoma brucei, der als tierpathogener Erreger aufgrund seiner Verwandtschaft zu den menschenpathogenen Trypanosomen als Modell zum Studium der menschlichen Schlafkrankheit dient. Ebenfalls wirksam ist die Verbindung 1 bei Infektion mit dem menschenpathogenen Erreger Trypanosoma rhodesiense.

Im Vergleich zum bekannten Pentamidin-Isethionat wurde allerdings eine geringfügige schwächere Wirksamkeit der Verbindung 1 gegenüber Trypanosma brucei und Trypanosoma rhodesiense festgestellt. Nur sehr schwach wirksamer war die Verbindung 1 gegenüber Trypanosoma vivax und Trypanosoma evansi. Bei Infektionen mit Trypanosoma congolense war die Verbindung 1 wirkungslos.

Die Ergebnisse der Testungen zeigen, daß das durch die neuartige pharmazeutische Formulierung gezeigte Wirkungsspektrum identisch ist mit dem von Pentamidin-Diisethionat. Sie zeigt sich bei einigen Trypanosomenspecies etwas schwächer wirksam. Trotzdem stellte die neuartige Zubereitung eine deutliche Verbesserung bestehender Therapiemöglichkeiten dar, da anders als beim Pentamidin-Diisethionat, dieses Medikament nicht parenteral appliziert werden muß. Wegen des Wegfalls der durch die Applikationsform bedingten Nebenwirkungen, besitzt die neuartige pharmazeutische Zubereitung den Vorteil, daß erstmals auch in Gebieten mit schlechter medizinischer Versorgung flächendeckend, Therapie und Prophylaxe gegen Trypanosomeninfektionen betrieben werden kann. Zusätzlich besteht die Möglichkeit, Patienten zu behandeln, die eine zentralnervöse Manifestation einer Trypanosomeninfektion zeigen, ohne die gravierenden, teilweise tödlich verlaufenden, Nebenwirkungen der bisher hier eingesetzten arsenhaltigen Therapeutika in Kauf zu nehmen.

Zum Nachweis der pharmazeutischen Wirksamkeit gegenüber Leishmanien wurde der nachfolgend wiedergegebene Test durchgeführt.

Der Test wurde an Goldhamstern vorgenommen, die mit Leishmania donovani infiziert wurden. Die jweils zu testenden Substanzen wurden in unterschiedlichen Dosierungen subcutan appliziert und deren Wirkamkeit der Wirksamkeit des bekannten Pentamidin-Isethionats gegenüber gestellt. Als Kontrollgruppen dienten infizierte, nicht behandelte Tiere.

Nach 8 Tagen wurde die Anzahl der Leishmanien pro Leberzellkern und Lebergewicht nach dem Modell nach Stauber et al J. Protozool. 5, 269 - 273 (1958) bestimmt.

In der Tabelle 7 sind nähere Einzelheiten des Testes am Beispiel der Verbindung 1 (Formel A, Ausführungsbeispiel 1) wiedergegeben.

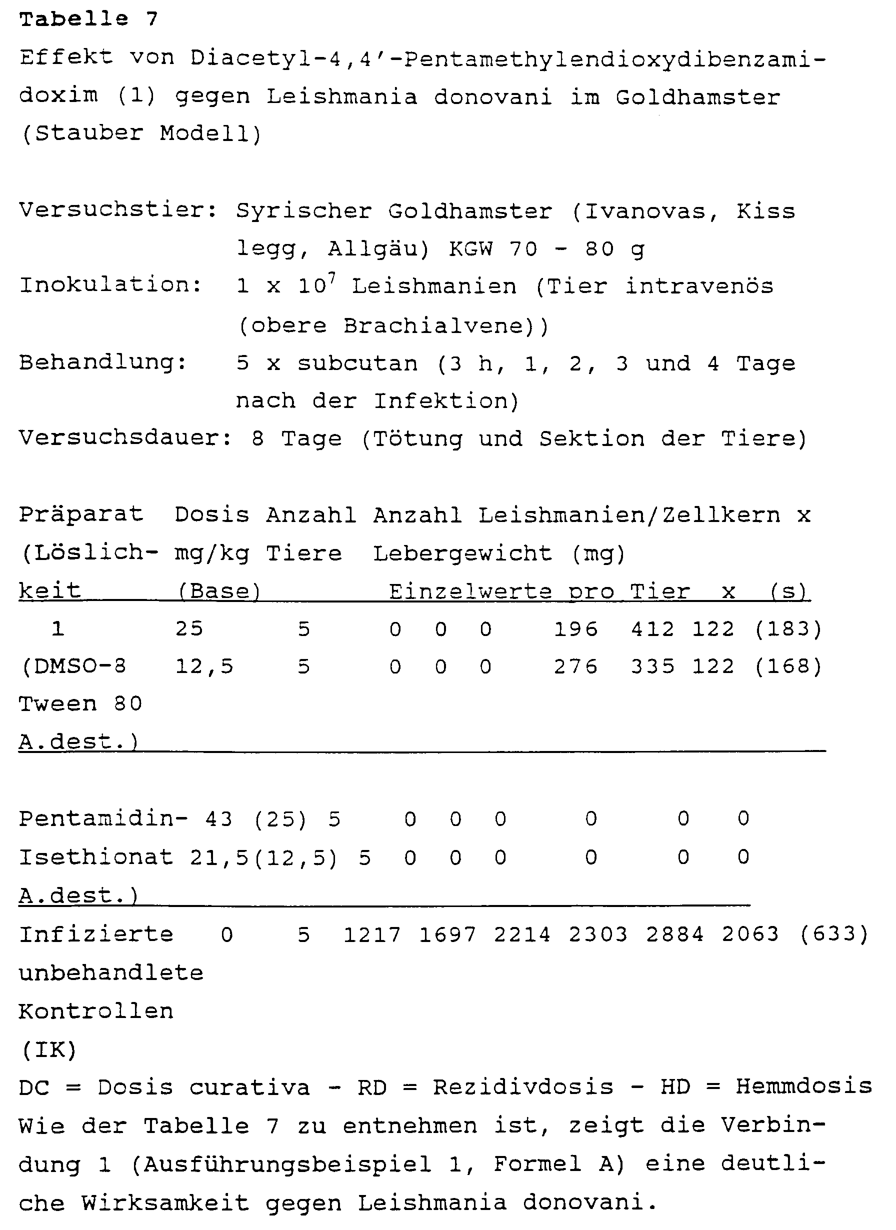

## Patentansprüche

1. Pharmazeutische Zubereitung zur oralen Applikation enthaltend mindestens einen Wirkstoff der mindestens eine Amidin-Gruppe aufweist und übliche Träger und Hilfsstoffe,
dadurch **gekennzeichnet,** daß die Zubereitung einen Pentamidin-Wirkstoff der allgemeinen Formel I enthält, 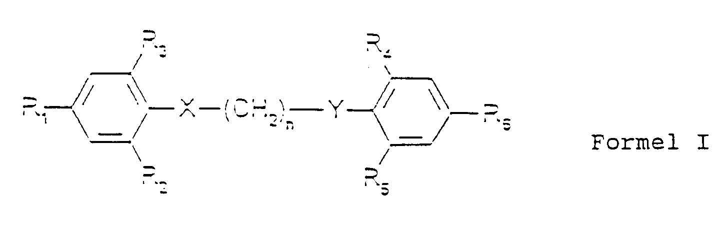 wobei in der Formel I
R₁ und R₆ gleich oder verschieden sind und aus folgender gruppe ausgewählt sind: 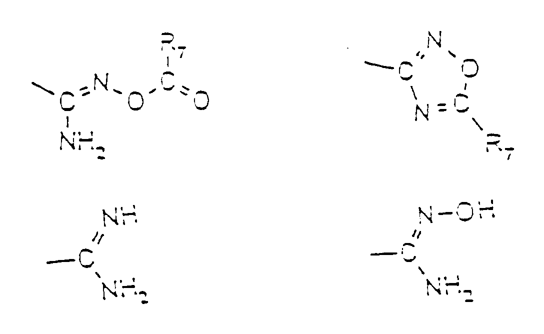
R₇ Wasserstoff, ein Alkylrest und/oder ein Arylrest;
R₂, R₃, R₄ und R₅ gleich oder verschieden sind und Wasserstoff, -No₂, Halogen und/oder eine OR₈-Gruppierung;
R₈ Wasserstoff und/oder eine Alkylgruppe;
X und Y gleich oder verschieden sind und Sauerstoff, Stickstoff oder Schwefel; und
n eine ganze Zahl zwischen 0 und 8 bedeutet
und/oder deren Salze mit der Maßgabe, daß in der allgemeinen Formel I nicht gleichzeitig R₁ und R₆ gleich oder verschieden sind und die Gruppierungen 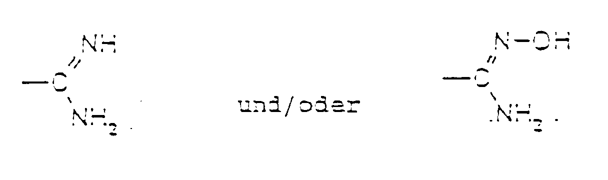 bedeuten, wenn n = 5 ist, R₂ bis R₅ Wasserstoff und wenn X und Y gleich sind und Sauerstoff bedeuten.

2. Pharmazeutische Zubereitung nach Anspruch 1,
dadurch gekennzeichnet, daß R₇, ein C₁₂-C₁₈-Alkylrest ist.

3. Pharmazeutische Zubereitung nach Anspruch 1 oder 2,
dadurch gekennzeichnet, daß R₇ eine C₁-C₄-Alkylgruppe, insbesondere eine CH₃-Gruppe ist.

4. Pharmazeutische Zubereitung nach mindestens einem der Ansprüche 1 bis 3,
dadurch gekennzeichnet, daß in der Formel I R₁ und R₆ gleich sind.

5. Pharmazeutische Zubereitung nach mindestens einem der Ansprüche 1 bis 4,
dadurch gekennzeichnet, daß in der allgemeinen Formel I n eine ganze Zahl zwischen 2 und 6 bedeutet.

6. Pharmazeutische Zubereitung nach mindestens einem der Ansprüche 1 bis 5,
dadurch gekennzeichnet, daß R₂, R₃, R₄ und/odr R₅ eine OCH₃-Gruppe bedeuten.

7. Pharmazeutische Zubereitung nach mindestens einem der Ansprüche 1 bis 6,
dadurch gekennzeichnet, daß die Zubereitung ein Gemisch von Wirkstoffen der allgmeinen Formel I enthält.

8. Pharmazeutische Zubereitung nach mindestens einem der Ansprüche 1 bis 7,
dadurch gekennzeichnet, daß die Zubereitung mindestens einen Wirkstoff der nachfolgend wiedergegebenen Formeln II bis IV 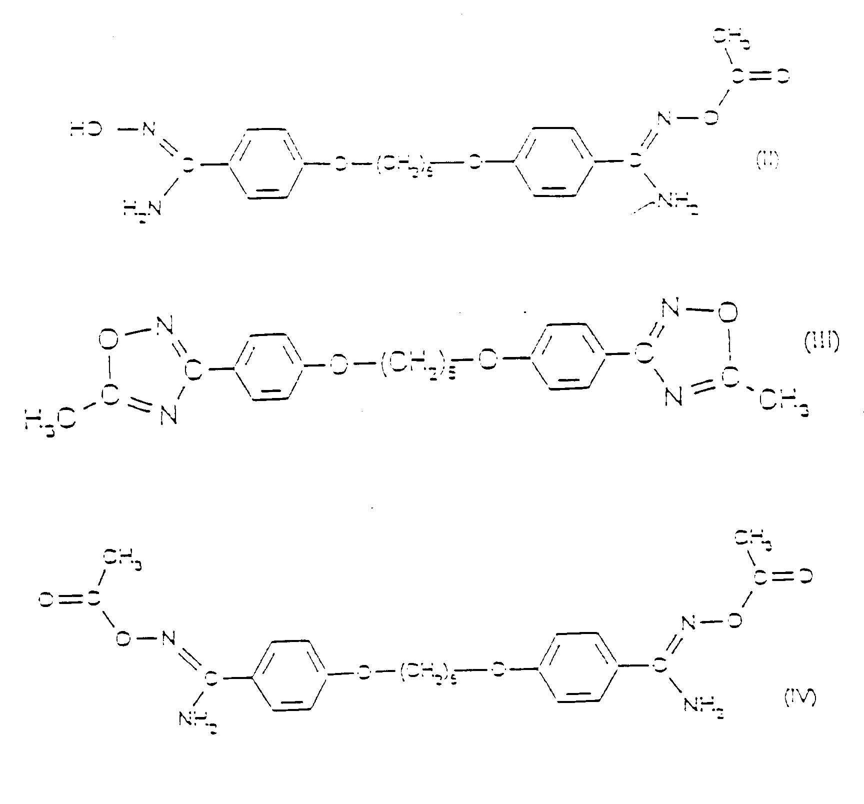 oder ein Salz dieser Wirkstoffe enthält.

9. Verwendung der pharmazeutischen Zubereitung nach einem der vorangehenden Ansprüche zur HerStellung eines Arzneimittels zur Prophylaxe und/oder zur Therapie von Pneumocystis carinii Pneumonie, insbesondere auch bei schweren Verlaufsformen mit extrapulmonarer Manifestition.

10. Verwendung der pharmazeutischen Zubereitung nach einem der vorangehenden Ansprüche zur Herstellung eines Arzneimittels zur Prophylaxe und/oder zur Therapie von tierischen oder menschlichen Trypanosomeninfektionen insbesondere auch bei Verlaufsformen mit zentralnervöser Manifestition.

11. Verwendung der pharmazeutischen Zubereitung nach einem der vorangehenden Ansprüche zur Herstellung eines Arzneimittels zur Prophylaxe und/oder zur Therapie von Leishmaniosen.

## Claims

1. Pharmaceutical preparation for oral administration comprising at least one active compound having at least one amidine group and customary carriers and auxiliaries,
characterized in that the preparation contains one active pentamidine compound of the general formula I 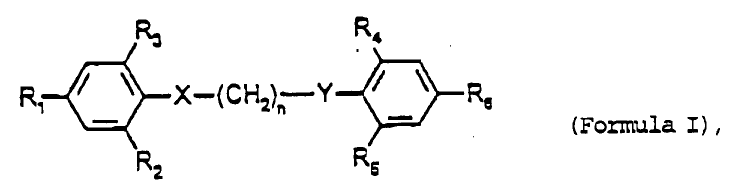 where in the formula I
R₁ and R₆ are identical or different and are selected from the following group: 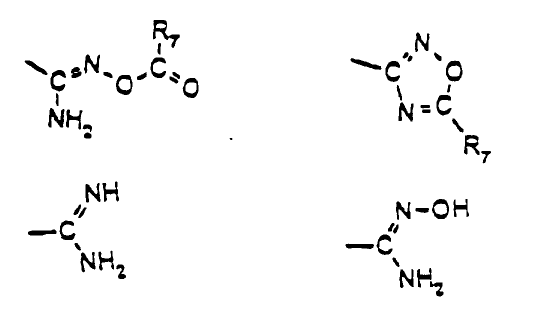
R₇ is hydrogen, an alkyl radical and/or an aryl radical;
R₂, R₃, R₄ and R₅ are identical or different and are each hydrogen, -NO₂, halogen and/or an OR₈ group;
R₈ is hydrogen and/or an alkyl group;
X and Y are identical or different and are each oxygen, nitrogen or sulphur; and
n is an integer between 0 and 8
and/or salts thereof with the proviso that in the general formula I R₁ and R₆ are not simultaneously identical or different and are the groups 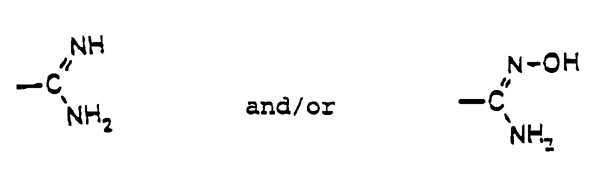 if n = 5, R₂ to R₅ are each hydrogen and if X and Y are identical and are each oxygen.

2. Pharmaceutical preparation according to Claim 1,
characterized in that R₇ is a C₁₂-C₁₈-alkyl radical.

3. Pharmaceutical preparation according to Claim 1 or 2,
characterized in that R₇ is a C₁-C₄-alkyl group, in particular a CH₃ group.

4. Pharmaceutical preparation according to at least one of Claims 1 to 3, characterized in that in the formula I R₁ and R₆ are identical.

5. Pharmaceutical preparation according to at least one of Claims 1 to 4
characterized in that in the general formula I n is an integer between 2 and 6.

6. Pharmaceutical preparation according to at least one of Claims 1 to 5,
characterized in that R₂, R₃, R₄ and/or R₅ are an OCH₃ group.

7. Pharmaceutical preparation according to at least one of Claims 1 to 6,
characterized in that the preparation comprises a mixture of active compounds of the general formula I.

8. Pharmaceutical preparation according to at least one of Claims 1 to 7,
characterized in that the preparation comprises at least one active compound of the formulae II to IV shown below 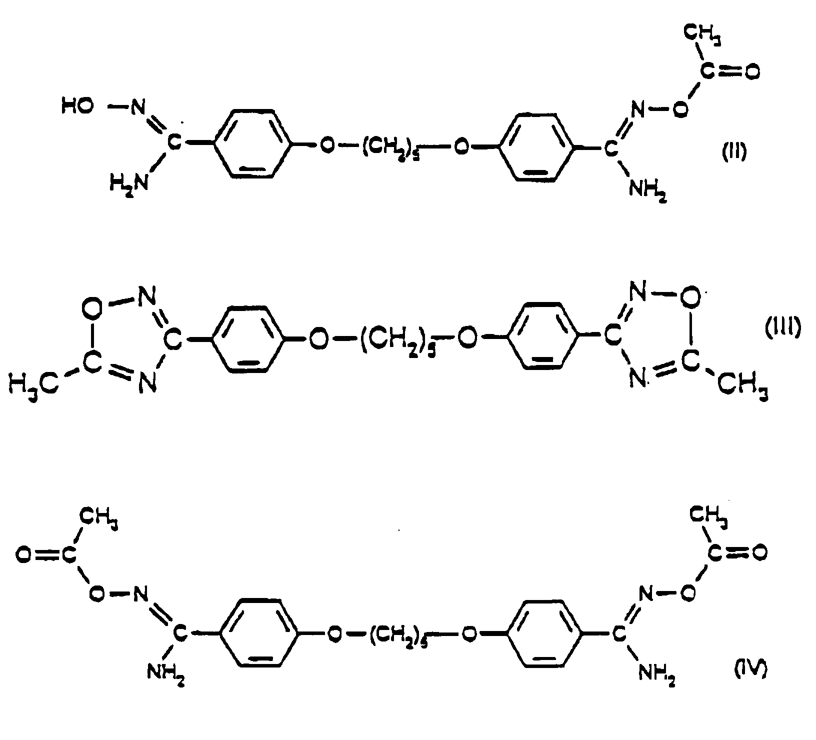 or a salt of these active compounds.

9. The use of the pharmaceutical preparation according to any of the preceding claims for preparing a medicament for the prophylaxis and/or for the therapy of Pneumocystis carinii pneumonia, in particular also for severe forms with extrapulmonary manifestation.

10. The use of the pharmaceutical preparation according to any of the preceding claims for preparing a medicament for the prophylaxis and/or for the therapy of trypanosomiases in animals and humans, in particular also for forms with manifestations in the central nervous system.

11. The use of the pharmaceutical preparation according to any of the preceding claims for preparing a medicament for the prophylaxis and/or for the therapy of leishmanioses.

## Revendications

1. Préparation pharmaceutique pour administration orale, renfermant au moins un principe actif qui présente au moins un groupe amidine, et les véhicules et agents auxiliaires habituels,
caractérisée en cc que la préparation contient un principe actif du type pentamidine de formule générale I 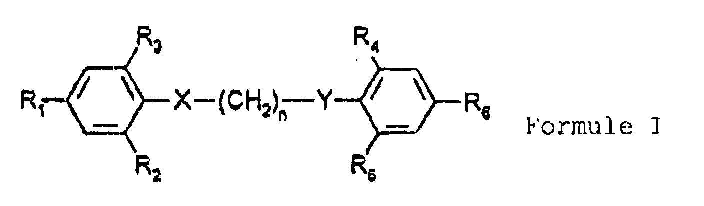 où, dans la. formule I,
R₁ et R₆ sont identiques ou différents, et sont choisis dans le groupe suivant: 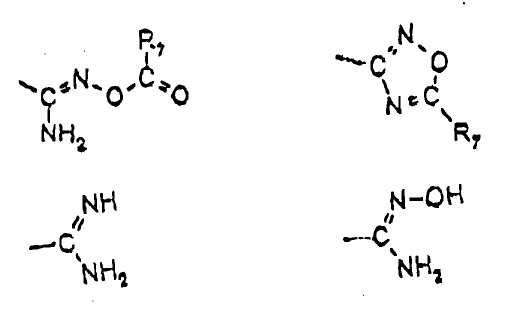
R₇ représente un atome d'hydrogène, un radical alkyle et/ou un radical aryle;
R₂, R₃, R₄ et, R₅ sont identiques ou différents, et représentent un atome d'hydrogène, un groupe -NO₂, un atome d'halogène et/ou un groupement OR₆;
R₈ représente un atome d'hydrogène et/ ou groupe alkyle;
X et Y sont identiques ou différents, et représentent un atome d'oxygène, d'azote ou de soufre; et
n représente un nombre entier entre 0 et 8;
et/ou ses sels, sous réserve de ne pas avoir en même temps, dans la formule générale I, R₁ et R₂ qui soient identiques ou différents et qui représentent les groupements 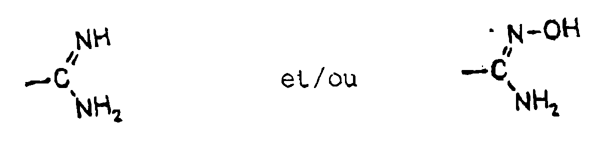 lorsque n = 5, et R₂ à R₅ qui représentent un atome d'hydrogène, lorsque X et Y sont identiques et représentent un atome d'oxygène.

2. Préparation pharmaceutique selon la revendication 1, caractérisée en ce que R₇ est un radical alkyle en C₁₂ -C₁₈.

3. Préparation pharmaceutique salon la revendication 1 ou 2,
caractérisée en ce que R₇ est un groupe alkyle en C₁ - C₄, en particulier un groupe CH₃.

4. Préparation pharmaceutique selon au moins l'une des revendications 1 à 3,
caractérisée en ce que, dans la formule I, R₁ et R₆ sont identiques.

5. Préparation pharmaceutique selon au moins l'une des revendications 1 à 4,
caractérisée en ce que, dans la formule générale I, n représente un nombre entier entre 2 et 6.

6. Préparation pharmaceutique selon au moins l'une des revendications 1 à 5,
caractérisée en ce que R₂, R₃, R₄ et/ou R₅ représentent un groupe OCH₃.

7. Préparation pharmaceutique selon au moins l'une des revendications 1 à 6,
caractérisée en ce que la préparation contient un mélange de principes actifs de formule générale I.

8. Préparation pharmaceutique, selon au moins l'une des revendications 1 à 7,
caractérisée en ce que la préparation contient au moins un principe actif possédant les formules II à IV représentées ci-après 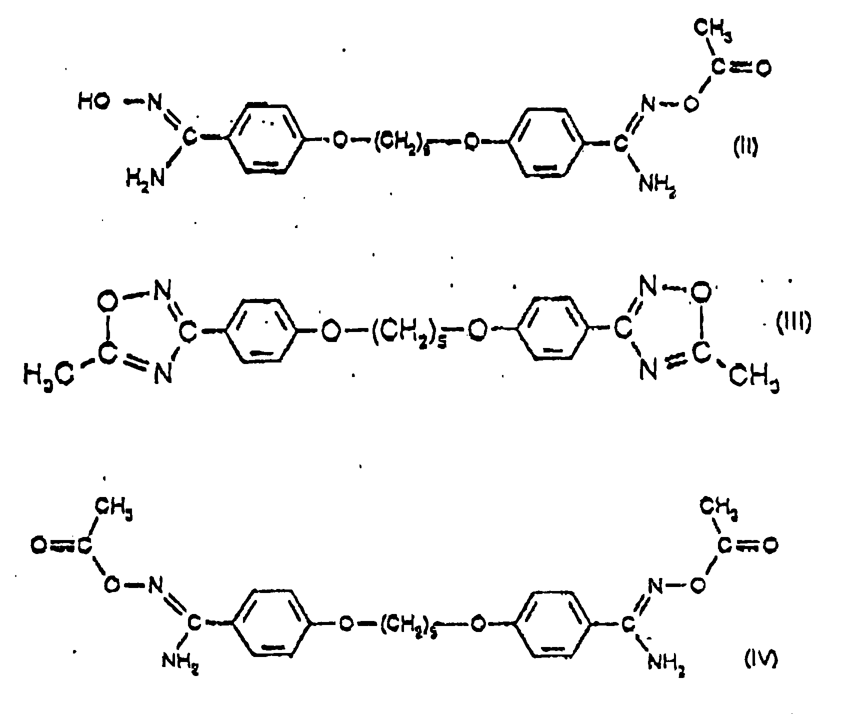 ou un sel de ce principe actif.

9. Utilisation de la préparation pharmaceutique selon l'une des revendications précédentes, pour fabriquer un médicament destiné à la prophylaxie et/ou à la thérapie de la pneumonie interstitielle à Pneumocystis carinii, en particulier également pour les formes d'évolution sérieuses comportant une manifestation extra-pulmonaire.

10. Utilisation de la préparation pharmaceutique selon l'une des revendications précédentes, pour fabriquer un médicament destiné à la prophylaxie et/ou à la thérapie dos infections animales ou humaines au trypanosome, en particulier également pour les formes d'évolution comportant une manifestation au niveau du système nerveux central.

11. Utilisation de la préparation pharmaceutique selon l'une des revendications précédentes, pour fabriquer un médicament destiné à la prophylaxie et/ou à la thérapie des leishmanioses.
